(19) Europäisches Patentamt / European Patent Office / Office européen des brevets

(11) **EP 4 253 547 A1**

(12) **EUROPEAN PATENT APPLICATION**
published in accordance with Art. 153(4) EPC

(43) Date of publication:
**04.10.2023  Bulletin 2023/40**

(21) Application number: **21898171.0**

(22) Date of filing: **29.11.2021**

(51) International Patent Classification (IPC):
*C12N 15/12* (2006.01)       *C12N 15/11* (2006.01)
*C12Q 1/6813* (2018.01)       *C12Q 1/6883* (2018.01)
*G01N 33/50* (2006.01)       *G01N 33/53* (2006.01)

(52) Cooperative Patent Classification (CPC):
**C07K 14/435; C12N 15/11; C12Q 1/6813;
C12Q 1/686; C12Q 1/6869; C12Q 1/6876;
C12Q 1/6883; G01N 33/50; G01N 33/53**

(86) International application number:
**PCT/JP2021/043714**

(87) International publication number:
**WO 2022/114200 (02.06.2022 Gazette 2022/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  30.11.2020  JP 2020198348

(71) Applicant: **Kao Corporation**
**Chuo-ku**
**Tokyo 103-8210 (JP)**

(72) Inventors:
• **TAKADA, Naoto**
  **Haga-gun, Tochigi 321-3497 (JP)**
• **KUWANO, Tetsuya**
  **Haga-gun, Tochigi 321-3497 (JP)**
• **INOUE, Takayoshi**
  **Haga-gun, Tochigi 321-3497 (JP)**

(74) Representative: **Vossius & Partner**
**Patentanwälte Rechtsanwälte mbB**
**Siebertstraße 3**
**81675 München (DE)**

(54) **METHOD FOR DETECTING SEVERITY OF ATOPIC DERMATITIS**

(57)    Provided are a marker for detecting the severity of atopic dermatitis and a method for detecting the severity of atopic dermatitis using the marker.

**Description**

Field of the Invention

The present invention relates to a marker for detecting the severity of atopic dermatitis and a method for detecting the severity of atopic dermatitis using the marker.

Background of the Invention

Atopic dermatitis (hereinafter, also referred to as "AD") is an eczematous skin disease that develops mainly in subjects having atopic predispositions. Typical symptoms of AD are chronic and recurrent itching, eruption, erythema, and so on that occur bilaterally and symmetrically, as well as parakeratosis, an impaired barrier function, dry skin, and so on. AD mainly occurs in infants and shows remission tendency with growth, but adult or intractable atopic dermatitis are also increasing in recent years. It is known that in AD, varieties of symptoms and phenotypes are formed due to complex involvement of various causes of the disease, and exacerbations and remissions are repeated (Non Patent Literature 1). For example, it has been reported that when moisturizing is not continued after induction of remission with a drug foe external use, symptoms are exacerbated in about 40% of AD patients within 14 days and about 60% of AD patients within 28 days (Non Patent Literature 2). Accordingly, in treatment of AD, it is necessary to correctly grasp the severity of AD including varieties of symptoms and phenotypes.

As a conventional method for assessing the severity of AD, assessment based on observations with the naked eye of a physician is mentioned. There are various observation items, such as dry symptoms, erythema, scales, papules, excoriation, edemas, adhesion of eschar, tiny blisters, erosions, and prurigo nodule. As indicators of scoring these observations, Eczema Area and Severity Index (EASI) and Severity SCORing of Atopic Dermatitis (SCORAD) are known. In addition, there is also a method of acquiring objective numerical values for symptoms associated with AD using an apparatus such as a high performance camera or probe. In contrast, assessment of AD by the patients themselves based on observations with the naked eye and awareness through touch is also performed, and as indicators of scoring for assessment, Patient Oriented Eczema Measure (POEM), Patient Oriented SCORAD (PO-SCORAD), and Visual Analog Scaling (VAS) are known.

However, in the various conventional methods for assessing the severity of AD, the assessment points are different from each other, and therefore it cannot always be said that the true severity of AD is correctly assessed when the assessment is performed by only one of the methods, and it is desirable to judge the severity of AD comprehensively based on the assessment results by multiple methods. On the other hand, from the viewpoint of the necessity of observations by a physician, the cost and availability of measuring equipment, or the burden on the patients themselves accompanied by self-assessment, the actual situation is that it is difficult to perform multiple conventional methods for assessing the severity of AD.

In recent years, it has been proposed to assess the pathological condition of AD using not only the phenotype appearing in observation and awareness but also the endotype of a pathobiological mechanism and to use it as a help for selecting the optimal treatment. That is, there is a possibility that AD patients have similar phenotypes that are caused by different molecular mechanisms. It is being believed that subdivision of pathological conditions of AD patients by combining the phenotype and the endotype leads to the optimal treatments suitable for individual patients. Presently, in order to objectively understand the pathological conditions of a disease or to understand the pathological conditions with considering the endotype, genes or their expression products contained in, for example, skin biopsy, blood or a horny cell layer, or the presence of specific cell types (which may be collectively referred to as biomarkers) are often used. Conventionally, as biomarkers for assessing the presence or absence or the severity of AD, blood peripheral blood eosinophil count, serum total IgE level, lactate dehydrogenase (LDH) level, serum thymus and activation-regulated chemokine (TARC), and squamous cell carcinoma antigen 2 (SCCA2), and so on have been proposed (Non Patent Literatures 3 and 4). However, the accuracy of these biomarkers is not necessarily sufficient.

In recent years, techniques to examine the present and also future physiological conditions in the human body by analysis of nucleic acid such as DNA and RNA in a biological specimen have been developed. Nucleic acid derived from a living body can be extracted from body fluids such as blood, secretions, tissues, and so on. Furthermore, recently, it has been reported that RNA included in skin surface lipids (SSL) can be used as a sample for biological analysis (Patent Literature 1). It has been also reported that a marker gene of atopic dermatitis can be detected from SSL (Patent Literature 2) .

Patent Literature 1: WO 2018/008319
Patent Literature 2: JP-A-2020-074769
Non Patent Literature 1: Kato, et al., The Japanese Journal of Dermatology, 2018, 128: 2431-2502

Non Patent Literature 2: Lin, et al., Adv. Ther., 2017, 34: 2601-2611
Non Patent Literature 3: Sugawara, et al., Allergy, 2002, 57: 180-181
Non Patent Literature 4: Ohta, et al., Ann. Clin. Biochem., 2012, 49: 277-284

Summary of the Invention

The present invention provides a marker for detecting the severity of atopic dermatitis, the marker comprising at least one selected from the group consisting of the following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes.

In addition, the present invention provides a method for detecting the severity of atopic dermatitis in a subject, the method comprising measuring the expression level of the marker for detecting the severity of atopic dermatitis in the subject.

In addition, the present invention provides use of at least one selected from the group consisting of the following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and the expression products of the genes as a marker for detecting the severity of atopic dermatitis, or for producing a marker for detecting the severity of atopic dermatitis.

Detailed Description of the Invention

All patent literatures, non-patent literatures, and other publications cited herein are hereby incorporated by reference in their entirety.

In the present specification, the terms "nucleic acid" and "polynucleotide" mean DNA or RNA. The term "DNA" includes all of cDNA, genomic DNA, and synthetic DNA, and the term "RNA" includes all of total RNA, mRNA, rRNA, tRNA, non-coding RNA, and synthetic RNA.

In the present specification, the term "gene" encompasses double-stranded DNA including human genomic DNA and also single-stranded DNA (positive strand) including cDNA, single-stranded DNA (complementary strand) having a sequence complementary to the positive strand, and fragments thereof and means that some biological information is included in the sequence information of nucleotides constituting the DNA. In addition, the term "gene" in the present specification encompasses not only the "gene" represented by a specific nucleotide sequence but also a congener (that is, homologue or orthologue), a mutant such as a genetic polymorphism, and a derivative thereof.

In the present invention, the "expression product" of a gene conceptually encompasses the transcription product and translation product of a gene. The "transcription product" is RNA resulting from transcription of a gene (DNA), and the "translation product" means protein encoded by a gene to be translated and synthesized based on the RNA.

In the present specification, the "skin surface lipids (SSL)" are a lipid-soluble fraction present on the surface of the skin and is also referred to as sebum. In general, SSL mainly contain secretions secreted from exocrine glands such as sebaceous glands in the skin and is present on the skin surface in a form of a thin layer covering the skin surface.

In the present specification, the term "skin" is a general term for regions including tissues such as horny cell layers, epidermis, dermis, and hair follicles, as well as sweat glands, sebaceous glands, and other glands, unless otherwise specified.

In the present specification, the "atopic dermatitis (also referred to as "AD")" indicates a disease that repeats exacerbation and remission and has pruritic eczema as a main pathogen, and many of the patients thereof are said to have atopic predispositions. Examples of the atomic predisposition include i) family history and medical history (one or more diseases of bronchial asthma, allergic rhinitis/conjunctivitis, and atopic dermatitis) and ii) predispositions being likely to produce IgE antibodies.

In the present specification, the "severity" of atopic dermatitis (AD) indicates the level of severity of AD symptoms, not presence or absence of AD, and includes not only rough classification such as mild, moderate, and severe but also classification by minute differences. The "severity" of AD can be determined based on, for example, known various assessment scores for assessing AD symptoms. In the present specification, the assessment scores are referred to as "scores associated with the severity of atopic dermatitis (AD)". Examples of the score associated with the severity of AD include the EASI score and the POEM score associated with the systemic eruption by AD, the VAS score for itching of the skin by AD, the VAS score for skin dryness by AD (Guideline for the management of atopic dermatitis, published by The Japanese Dermatological Association, The Journal of Dermatology: 128(12), 2431-2502 (2018)), and the erythema index associated with facial erythema by AD (see JP-A-2018-23756 and Dawson, et al., Phys. Med. Biol., 25 (1980)). Alternatively, a score determined by comprehensively assessing two or more selected from these scores and indexes may be used. As the "severity", the score itself according to the severity of AD may be used as the level of severity of AD symptoms.

In the present specification, the "detection" of the severity of AD can also be expressed by another term such as test, measurement, judgement, or assessment support. The term "detection", "test", "measurement", "judgement", or "assessment" of the severity of AD in the present specification does not include diagnosis of the severity of AD by a physician.

The present invention relates to a marker for detecting the severity of atopic dermatitis and a method for detecting the severity of atopic dermatitis using the marker.

The marker for detecting the severity of atopic dermatitis of the present invention provides an indicator for detecting the severity of atopic dermatitis. The severity of an atopic dermatitis patient can be easily detected by using the marker, eventually, it is possible to provide a correct grasp of the pathological conditions of a patient and optimal treatment for the patient.

(1. Marker for detecting severity of atopic dermatitis)

A biomarker reflecting the severity of AD has been required. Existing markers for assessing the presence or absence or the severity of AD have been found mainly based on population analysis, i.e., comparison between groups belonging to different severity (for example, a patient group and a normal group, or a severe group and a mild group). However, existing markers found by such population analysis may not always reflect minute differences in severity in each group, and it is difficult to assess the severity of AD in detail by these existing markers. If the severity of an AD patient can be detected more accurately, the pathological conditions of the patient can be correctly grasped, eventually, it is possible to provide optimal treatment for the patient.

The present inventors found that a minute difference in the severity of an AD patient is reflected in the expression level of a specific gene in the patient. As shown in examples below, examined was the relationship between scores associated with the severity of AD in a subject based on existing various indicators and expression levels of various genes in the subject. As a result, genes of which the expression levels have a positive correlation or a negative correlation with the scores associated with the severity of AD in the subject were found. These genes or expression products thereof reflect the differences in AD severity in detail and can be used as markers for detecting the severity of AD in a subject. For example, it is possible to detect in detail how the degree of severity of AD in the subject is or to detect whether the severity is exacerbated or remitting by using an expression level of such a gene or the expression product of the gene as an indicator.

Accordingly, in an aspect, the present invention provides a marker for detecting the severity of AD. In an embodiment, the marker for detecting the severity of AD provided by the present invention (hereinafter, also referred to as marker of the present invention) can be used not only as a marker for roughly classifying the AD severity in a subject into, for example, mild, moderate, and severe as in existing markers but also as a marker for distinguishing more minute differences in severity. Furthermore, it is possible to detect a change (for example, exacerbation or remission) in the AD severity in a subject by comparing the severity of AD in the subject detected by the marker of the present invention at different time points.

The marker of the present invention can include at least one selected from the group consisting of 7 genes shown in Table 1A below and 15 genes shown in Table 1B below, 22 genes in total, and expression products thereof. The names (Gene Symbols) and Gene IDs of genes shown in Tables 1A and 1B conform to the Official Symbol and Gene ID described in the NCBI

([www.ncbi.nlm.nih.gov/]). Hereinafter, the genes and expression products shown in Table 1A are also collectively referred to as markers of Table 1A, and the genes and expression products shown in Table 1B are also collectively referred to as markers of Table 1B. The marker of the present invention may be the gene shown in Table 1A or 1B below, or an expression product thereof, or a combination thereof. In an embodiment, the marker of the present invention is a nucleic acid marker such as the DNA of the gene or the RNA as a transcription product thereof. In another embodiment, the marker of the present invention is a protein marker as a translation product of the gene. The marker of the present invention is preferably a nucleic acid marker.

[Table 1]

| A | |
| --- | --- |
| Gene Symbol | Gene ID |
| ADAM 15 | 8751 |
| CIZ1 | 25792 |
| LYNX1 | 66004 |
| ODC1 | 4953 |

(continued)

| A | | | |
|---|---|---|---|
| Gene Symbol | Gene ID | | |
| PSME2 | 5721 | | |
| SETD1B | 23067 | | |
| TWF1 | 5756 | | |
| B | | | |
| Gene Symbol | Gene ID | Gene Symbol | Gene ID |
| AGR2 | 10551 | LSM10 | 84967 |
| ALPK1 | 80216 | PDK4 | 5166 |
| APOD | 347 | PLXNC1 | 10154 |
| ATG16L2 | 89849 | SASH3 | 54440 |
| CSNK1D | 1453 | SLC12A6 | 9990 |
| FASN | 2194 | TSC22D3 | 1831 |
| GSK3A | 2931 | VSIR | 64115 |
| ITPKB | 3707 | | |

The genes shown in Tables 1A and 1B encompass a gene consisting of the nucleotide sequences registered in the NCBI and also a gene consisting of sequences substantially identical to the registered sequences as long as the genes itself or an expression product derived therefrom functions as a marker for detecting the AD severity. Here, the substantially identical sequence means, for example, a sequence having an identity of 90% or more, preferably 95% or more, more preferably 98% or more, and further preferably 99% or more with the nucleotide sequence of the gene, for example, when the search is performed using homology calculation algorithm NCBI BLAST under conditions: expectation value = 10; a gap acceptable; filtering = ON; match score = 1; and mismatch score = -3.

As shown in examples described later, the expression levels of the markers of Table 1A showed a positive correlation with scores associated with the severity of AD. That is, the markers of Table 1A are positive markers of which the expression levels show a positive correlation with the severity of AD. In contrast, the expression levels of the markers of Table 1B showed a negative correlation with scores associated with the severity of AD. That is, the markers of Table 1B are negative markers of which the expression levels show a negative correlation with the severity of AD. The present invention may use either the former positive markers or the latter negative markers or may use a combination of both.

In a preferable embodiment, the marker of the present invention is a marker for detecting the severity of systemic eruption by AD, for example, a marker that can detect the severity of AD corresponding to the EASI score. The marker includes at least one selected from the group consisting of the following genes: CIZ1, ADAM15, SETD1B, and TWF1, and the expression products of the genes.

These markers are positive markers included in Table 1A. The expression levels of the positive markers show a positive correlation with the severity of systemic eruption by AD, for example, with the EASI score.

In another preferable embodiment, the marker of the present invention is a marker for detecting the severity of systemic eruption by AD, for example, a marker that can detect the severity of AD corresponding to the POEM score. The marker includes at least one selected from the group consisting of the following genes: LYNX1 and PSME2, and the expression products of the genes.

These markers are positive markers included in Table 1A. The expression levels of the positive markers show a positive correlation with the severity of systemic eruption by AD, for example, with the POEM score.

In another preferable embodiment, the marker of the present invention is a marker for detecting the severity of itching of the skin by AD, for example, a marker that can detect the severity of itching of the skin by AD corresponding to the VAS score of itching of the skin. The marker includes at least one selected from the group consisting of the following genes: ALPK1, ATG16L2, CSNK1D, GSK3A, LSM10, SASH3, and VSIR, and the expression products of the genes.

These markers are negative markers included in Table 1B. The expression levels of the negative markers show a negative correlation with the severity of itching of the skin by AD, for example, with the VAS score of itching of the skin.

In another preferable embodiment, the marker of the present invention is a marker for detecting the severity of skin dryness by AD, for example, a marker that can detect the severity of skin dryness by AD corresponding to the VAS score of skin dryness. The marker includes at least one selected from the group consisting of the following genes: TSC22D3, PLXNC1, and SLC12A6, and the expression products of the genes.

These markers are negative markers included in Table 1B. The expression levels of the negative markers show a negative correlation with the severity of skin dryness by AD, for example, with the VAS score of skin dryness.

In another preferable embodiment, the marker of the present invention is a marker for detecting the severity of facial erythema by AD, for example, a marker that can detect the severity of facial erythema by AD corresponding to the erythema index. The marker includes at least one selected from the group consisting of the following genes: ODC1, AGR2, FASN, APOD, ITPKB, and PDK4, and the expression products of the genes.

These markers include positive markers included in Table 1A and negative markers included in Table 1B. The expression levels of the positive markers show a positive correlation with the severity of facial erythema by AD, for example, with the erythema index. In contrast, the expression levels of the negative markers show a negative correlation with the severity of facial erythema by AD, for example, with the erythema index.

The marker of the present invention can be prepared from a biological specimen collected from a subject, for example, cells, tissues (such as biopsy), body fluids (for example, body fluids such as tissue exudate, blood, and serum and plasma prepared from blood), organs, skin, urine, saliva, sweat, horny cell layers, skin surface lipids (SSL), stool, and hairs, by a usual method. For example, nucleic acid or protein can be prepared from a biological specimen using a commercially available kit. Preferably, the marker of the present invention is a nucleic acid marker, and preferable examples of the nucleic acid prepared from a biological specimen include DNA such as genomic DNA and RNA such as mRNA.

Examples of the subject from which a biological specimen containing the marker of the present invention is collected include a mammal including a human and non-human mammal, and the subject is preferably a human. When the subject is a human, the gender, age, race, and so on are not particularly limited, and humans from infants to elders can be included. Examples thereof include a person who has AD, a person who needs or wishes to detect the severity of AD, and a person who needs or wishes to detect a change in the severity of AD.

The marker of the present invention is more preferably nucleic acid or protein prepared from SSL of a subject and further preferably mRNA. The part of the skin from which SSL is collected is not particularly limited, and examples thereof include the skin of any part of the body, such as head, face, neck, trunk, and limbs. Parts with high sebum secretion, for example, the skin of the head or face is preferable, and the skin of the face is more preferable. The part of the skin from which SSL is collected may be an eruption area with AD or a non-eruption area without AD, but preferred is an eruption area or a non-eruption area near an eruption area. Here, the area near an eruption area indicates an area within 10 cm adjacent to the eruption area.

In the collection of SSL from skin of a subject, any means used for collection or removal of SSL from skin can be employed. Preferably, an SSL-absorptive material or an SSL-adhesive material described later, or a tool for scraping SSL from skin can be used. The SSL-absorptive material or SSL-adhesive material is not particularly limited as long as it has affinity to SSL, and examples thereof include polypropylene and pulp. More detailed examples of the procedure of collection of SSL from skin include a method of absorbing SSL into a sheet-like material such as oil blotting paper or an oil blotting film, a method of allowing SSL to adhere to a glass plate, tape, or the like, and a method of collecting SSL by scraping with a spatula, scraper, or the like. In order to improve the absorption of SSL, an SSL-absorptive material impregnated with a highly lipid-soluble solvent in advance may be used. On the other hand, if the SSL-absorptive material contains a highly water-soluble solvent or water, absorption of SSL is prevented, and therefore it is preferable that the content of the highly water-soluble solvent or water is low. The SSL-absorptive material is preferably used in a dry state.

The collected SSL may be immediately subjected to a step of extracting the nucleic acid or protein described below or may be stored before use in the step of extracting the nucleic acid or protein. When stored, SSL are preferably stored under low temperature conditions. The temperature conditions for storing SSL may be 0°C or less, preferably -20±20°C to -80±20°C, more preferably - 20±10°C to -80±10°C, further preferably -20±20°C to - 40±20°C, further preferably -20±10°C to -40±10°C, further preferably -20±10°C, and further preferably -20±5°C. The storage period of SSL is not particularly limited and is preferably 12 months or less, for example, 6 hours or more and 12 months or less, more preferably 6 months or less, for example, one day or more and 6 months or less, and further preferably 3 months or less, for example, 3 days or more and 3 months or less.

In the extraction of the nucleic acid or protein from the collected SSL, a method that is usually used in extraction or purification of nucleic acid or protein from a biological specimen can be used. Examples of the method of extracting or purifying a nucleic acid include a phenol-chloroform method, an acid guanidinium thiocyanate-phenol-chloroform extraction (AGPC) method, a method using a column such as TRIzol (Registered Trademark), RNeasy (Registered Trademark), or QIAzol (Registered Trademark), a method using special magnetic particles coated with silica, a method using solid phase reversible immobilization magnetic particles, and extraction with a commercially available RNA extraction reagent such as ISOGEN. Extraction or purification of protein can use a commercially available protein extraction reagent such as QIAzol Lysis Reagent (QIAGEN N.V.) .

(2. Method for detecting severity of atopic dermatitis)

In another aspect, the present invention provides a method for detecting the severity of AD using the marker of the present invention described in the above paragraph 1. In the method for detecting the severity of AD by the present invention (hereinafter, referred to as the method of the present invention), the severity of AD in a subject is detected based on the expression level of the marker of the present invention in the subject. In an embodiment, the method of the present invention detects the severity of AD in a subject, i.e., how the degree of severity of the symptom is, using the expression level of the marker of the present invention as an indicator. Furthermore, it is possible to detect a change (for example, exacerbation or remission) in the AD severity in a subject by comparing the severity detected at different time points. Accordingly, in another embodiment of the method of the present invention, a change in the severity (for example, exacerbation or remission of the symptom) of AD in a subject is detected using the change in the expression level of the marker of the present invention as an indicator.

(2.1 Analysis of expression of marker)

The subject to be applied to the method of the present invention is same as the subject from which a biological specimen including the marker of the present invention described above is collected. In a preferred embodiment, the method of the present invention includes measurement of the expression level of the marker of the present invention in a biological specimen collected from a subject. The type of the biological specimen is as described above and is preferably SSL. In an embodiment, the method of the present invention may further include collection of SSL of the subject. The procedure of collecting SSL and the procedure of extracting a marker from the SSL are as described above.

The expression level of the marker of the present invention can be measured according to a quantitative measurement method of nucleic acid or protein that is commonly used in the field. The expression level of a marker to be measured may be the expression level based on the absolute amount of a target marker in the biological specimen or may be a relative expression level with respect to the expression level of another standard material, the total nucleic acids, or the total proteins.

For example, the expression level of a nucleic acid marker may be measured according to a procedure of gene expression analysis that is commonly used in the field. Examples of the means of the gene expression analysis include methods for quantitatively measuring nucleic acid or amplification product thereof, such as PCR, multiplex PCR, real-time PCR, hybridization (DNA chip, DNA microarray, dot-blot hybridization, slot-blot hybridization, northern blot hybridization, and so on), sequencing, and chromatography. When the nucleic acid is RNA, it is preferable to convert the RNA into cDNA by reverse transcription and then perform quantitative measurement by the method above.

The expression level of a protein marker can be measured using a protein quantitative measurement method that is commonly used in the field, for example, immunoassay (for example, western blotting, ELISA, and immunostaining), fluorescence method, electrophoresis, protein chip, chromatography, mass spectrometry (for example, LC-MS/MS and MALDI-TOF/MS), 1-hybrid method (PNAS, 100, 12271-12276 (2003)), and 2-hybrid method (Biol. Reprod., 58, 302-311 (1998)). Alternatively, the expression level of the marker of the present invention may be measured by measuring a molecule that interacts with the nucleic acid or protein as the marker of the present invention. Examples of the molecule that interacts with the marker of the present invention include DNA, RNA, protein, polysaccharides, oligosaccharides, monosaccharides, lipids, fatty acids, and phosphorylated products, alkylated products, and glycosylated products thereof, and complexes of any of the above.

The marker that is used in the method of the present invention is preferably RNA derived from SSL. In this case, the expression level of the RNA included in SSL is measured. The expression levels of the RNA derived from SSL is preferably measured by converting RNA extracted from SSL into cDNA by reverse transcription and then quantitatively measuring the cDNA or amplification product thereof by the means above.

The reverse transcription of RNA may use a primer targeting specific RNA to be analyzed, but it is preferable to use a random primer for more comprehensive nucleic acid storage and analysis. The reverse transcription can use a common reverse transcriptase or reverse transcription reagent kit. Suitably, a reverse transcriptase or reverse transcription reagent kit with high accuracy and efficiency is used, and examples thereof include M-MLV Reverse Transcriptase and a modified product thereof and a commercially available reverse transcriptase or reverse transcription reagent kit, such as PrimeScript (Registered Trademark) Reverse Transcriptase series (TAKARA BIO INC.), SuperScript (Registered Trademark) Reverse Transcriptase series (Thermo Fischer Scientific Inc.), SuperScript (Registered Trademark) III Reverse Transcriptase, and SuperScript (Registered Trademark) VILO cDNA Synthesis kit (both, Thermo Fischer Scientific Inc.).

In the extension reaction in the reverse transcription, the temperature is preferably adjusted to 42°C±1°C, more preferably 42°C±0.5°C, and further preferably 42°C±0.25°C, and the reaction time is preferably adjusted to 60 minutes or more and more preferably from 80 to 120 minutes.

When the expression level of a nucleic acid marker is measured by PCR, RNA derived from a biological specimen is reverse-transcribed into cDNA as needed, and the DNA derived from the biological specimen is then amplified using a primer pair. In PCR, a primer pair targeting one specific DNA to be analyzed may be used to amplify the specific DNA only, or multiple primer pairs may be used to amplify multiple specific DNAs simultaneously. The PCR is preferably multiplex PCR. The multiplex PCR is a method using multiple primer pairs simultaneously in a PCR reaction system to simultaneously amplify multiple gene regions. The multiplex PCR can be carried out using a commercially available kit (for example, Ion AmpliSeq Transcriptome Human Gene Expression Kit, Life Technologies Japan Ltd.).

The temperature for the annealing and extension reaction in the PCR varies depending on the primers used and thus cannot be generalized but is preferably 62°C±1°C, more preferably 62°C±0.5°C, and further preferably 62°C±0.25°C when the multiplex PCR kit described above is used. Accordingly, in the PCR, annealing and extension reaction are preferably performed in one step. The time of the step of annealing and extension reaction can be adjusted depending on the size of the DNA to be amplified and so on and is preferably from 14 to 18 minutes. The conditions for degeneration reaction in the PCR can be adjusted depending on the DNA to be amplified and is preferably from 95°C to 99°C for from 10 to 60 seconds. The reverse transcription and PCR can be carried out at the temperature for the time period as above using a thermal cycler that is commonly used for PCR.

The reaction product obtained by the PCR is preferably purified by size separation of the reaction product. The size separation can separate the target PCR reaction product from the primers and other impurities contained in the PCR reaction liquid. The size separation of DNA can be performed with, for example, a size separation column, a size separation chip, or magnetic beads that can be used for size separation. Preferred examples of the magnetic beads that can be used for size separation include Solid Phase Reversible Immobilization (SPRI) magnetic beads such as Ampure XP.

The purified PCR reaction product may be further subjected to treatment necessary for performing subsequent quantitative analysis. For example, in order to sequence the DNA, the purified PCR reaction product may be prepared into an appropriate buffer solution, the PCR primer region included in the PCR-amplified DNA may be cleaved, or an adaptor sequence may be further added to the amplified DNA. For example, a library for quantitative analysis can be prepared by preparing the purified PCR reaction product into a buffer solution, subjecting the amplified DNA to PCR primer sequence removal and adaptor ligation, and amplifying the resulting reaction product as needed. These operations can be performed, for example, using 5×VILO RT Reaction Mix attached to SuperScript (Registered Trademark) VILO cDNA Synthesis kit (Life Technologies Japan Ltd.) and 5×Ion AmpliSeq HiFi Mix and Ion AmpliSeq Transcriptome Human Gene Expression Core Panel attached to Ion AmpliSeq Transcriptome Human Gene Expression Kit (Life Technologies Japan Ltd.) according to the protocol attached to each kit.

When the expression level of a nucleic acid marker is measured using real-time PCR, the RNA derived from a biological specimen is reverse-transcribed into cDNA as needed, PCR is then performed using primers labeled with a radioisotope (RI), a fluorescent material, or the like in advance, and the produced labeled double-stranded DNA is detected or quantitatively measured.

When the expression level of a nucleic acid marker is measured by northern blot hybridization, for example, RNA derived a biological specimen is transferred onto a membrane according to a usual method, and probe DNA labeled with an RI, fluorescent material, or the like is then allowed to hybridize with the RNA. The expression level of the nucleic acid marker can be measured by detecting the signal derived from the label in the formed double strand of the labeled probe DNA and the RNA.

When the expression level of a nucleic acid marker is measured using a DNA microarray, for example, a microarray in which nucleic acid (cDNA or DNA) that specifically hybridizes with a target nucleic acid marker is immobilized on a support is used. The expression level of a nucleic acid marker in a biological specimen can be measured by binding nucleic acid (cDNA or cRNA) prepared from the biological specimen onto the microarray and detecting the label on the microarray.

The nucleic acid to be immobilized on the microarray may be any nucleic acid that hybridizes specifically with the target nucleic acid marker (i.e., substantially only the target nucleic acid marker) under stringent conditions, and may be nucleic acid including the complete sequence of the nucleic acid marker of the present invention or nucleic acid consisting of a partial sequence. Examples of the "partial sequence" include nucleic acid consisting of at least from 15 to 25 nucleotides. Here, the stringent conditions may be usually washing conditions of about "1×SSC, 0.1% SDS, 37°C", preferably about "0.5×SSC, 0.1% SDS, 42°C", and further preferably about "0.1×SSC, 0.1% SDS, 65°C". Stringent hybridization conditions are described in, for example, J. Sambrook, et al., Molecular Cloning: A Laboratory Manual, Third Edition, Cold Spring Harbor Laboratory Press (2001).

When the expression level of a nucleic acid marker is measured using sequencing, a next-generation sequencer (for example, Ion S5/XL system, Life Technologies Japan Ltd.) can be preferably used. The expression level of DNA or RNA can be measured based on the number of reads (read count) produced by sequencing.

When the expression levels of multiple nucleic acid markers are measured by sequencing, the above-mentioned read count can be used as the data of the expression level. Alternatively, for example, the RPM (reads per million mapped reads) value of the read count corrected for differences in the total number of reads between samples in the read count,

the logarithmic value (Log$_2$RPM value or Log$_2$(RPM + 1) value) of the RPM value, and the count value (normalized count value) corrected using DESeq2 (Love M.I., et al., Genome Biol., 2014) or its logarithmic value (Log$_2$ (normalized count + 1) value) can be used as the data of the expression level. Alternatively, as the data of the expression level, for example, fragments per kilobase of exon per million reads mapped (FPKM), reads per kilobase of exon per million reads mapped (RPKM), or transcripts per million (TPM), which are common as the quantitative values of RNA-seq, can be used.

The probe or primer that is used in measurement of a nucleic acid marker can be, for example, the primer for amplifying specifically the nucleic acid marker of the present invention or the probe for detecting specifically the nucleic acid marker. Here, the term "specifically" means that a nucleic acid can be recognized or detected such that a produced or detected substance derived from the marker of the present invention is substantially produced, for example, only the marker of the present invention is substantially detected in northern blotting, or only the marker of the present invention is substantially amplified in PCR. These probes or primers can be designed based on the nucleotide sequence of the nucleic acid marker.

As specific examples of the probe or primer, oligonucleotides consisting of the complete sequence or a partial sequence of the nucleic acid marker of the present invention or complementary strands thereof can be used. The "complementary strand" is not limited to a completely complementary sequence as long as it specifically recognizes the target marker, and may be a sequence preferably having a sequence identity of 80% or more, more preferably 90% or more, further preferably 95% or more, and further preferably 98% or more. The identity of a sequence can be determined by algorithm such as NCBI BLAST mentioned above.

Examples of the primer that is used in measurement of the nucleic acid marker are those capable of specific annealing and strand extension for a target nucleic acid marker and preferably having a strand length of 10 or more nucleotides, more preferably 15 or more nucleotides, and further preferably 20 or more nucleotides and preferably 100 or less nucleotides, more preferably 50 or less nucleotides, and further preferably 35 or less nucleotides.

Examples of the probe that is used in measurement of the nucleic acid marker are those capable of specific hybridization with a target nucleic acid marker and preferably having a strand length of 10 or more nucleotides, more preferably 15 or more nucleotides, and preferably 100 or less nucleotides, more preferably 50 or less nucleotides, and further preferably 25 or less nucleotides.

The probe or primer can be DNA or RNA and may be synthetic or naturally occurring. The probe to be used for hybridization is usually labeled one.

When the expression level of a protein marker is measured by immunoassay, for example, an antibody against the protein marker is brought into contact with a biological specimen, and the protein marker bound to the antibody may be quantitatively measured. For example, in western blotting, a primary antibody against the protein marker is used, the primary antibody is then labeled using a secondary antibody labeled with an RI, fluorescent material, enzyme, or the like, and then the expression level of the protein marker can be measured by measuring the signal derived from the label. The antibody against the protein marker may be a polyclonal antibody or a monoclonal antibody. These antibodies can be produced according to known methods.

(2.2 Detection of severity based on expression level of marker)

In an embodiment of the method of the present invention, the severity of AD in a subject is detected based on the expression level of the marker of the present invention derived from the subject (a marker of the present invention included in a biological specimen collected from the subject).

As described above, the markers of Tables 1A and 1B are markers of which the expression levels vary depending on the severity of AD. In more detail, the markers of Table 1A are positive markers of which the expression levels show a positive correlation with the severity of AD. In contrast, the markers of Table 1B are negative markers of which the expression levels show a negative correlation with the severity of AD. Accordingly, the severity of AD in a subject can be detected using the expression level of the positive marker or the negative marker as an indicator.

In a preferable example of the present embodiment, the severity of AD detected by the method of the present invention is the severity of systemic eruption by AD, for example, the severity of AD corresponding to the EASI score. The marker that is used in this detection is a marker for detecting the severity of systemic eruption by AD described above, for example, a marker that can detect the severity of AD corresponding to the EASI score. These markers are positive markers, and the expression levels thereof indicate a positive correlation with the severity of systemic eruption by AD, for example, with the EASI score. The severity of systemic eruption by AD in a subject can be detected using the expression level of the marker as an indicator.

In another preferable example of the present embodiment, the severity of AD detected by the method of the present invention is the severity of systemic eruption by AD, for example, the severity of AD corresponding to the POEM score. The marker that is used in this detection is a marker for detecting the severity of systemic eruption by AD described above, for example, a marker that can detect the severity of AD corresponding to the POEM score. These markers are positive markers, and the expression levels thereof indicate a positive correlation with the severity of systemic eruption by AD, for example, with the POEM score. The severity of systemic eruption by AD in a subject can be detected using the expression level of the marker as an indicator.

In another preferable example of the present embodiment, the severity of AD detected by the method of the present invention is the severity of itching of the skin by AD, for example, the severity of AD corresponding to the VAS score of itching of the skin. The marker that is used in this detection is a marker for detecting the severity of itching of the skin by AD described above, for example, a marker that can detect the severity of AD corresponding to the VAS score of itching of the skin. These markers are negative markers, and the expression levels thereof indicate a negative correlation with the severity of itching of the skin by AD, for example, with the VAS score of itching of the skin. The severity of itching of the skin by AD in a subject can be detected using the expression level of the marker as an indicator.

In another preferable example of the present embodiment, the severity of AD detected by the method of the present invention is the severity of skin dryness by AD, for example, the severity of AD corresponding to the VAS score of skin dryness. The marker that is used in this detection is a marker for detecting the severity of skin dryness by AD described above, for example, a marker that can detect the severity of AD corresponding to the VAS score of skin dryness. These markers are negative markers, and the expression levels thereof indicate a negative correlation with the severity of skin dryness by AD, for example, with the VAS score of skin dryness. The severity of skin dryness by AD in a subject can be detected using the expression level of the marker as an indicator.

In another preferable example of the present embodiment, the severity of AD detected by the method of the present invention is the severity of facial erythema by AD, for example, the severity of AD corresponding to the erythema index. The marker that is used in this detection is a marker for detecting the severity of facial erythema by AD described above, for example, a marker that can detect the severity of AD corresponding to the erythema index. When these markers are positive markers, the expression levels thereof indicate a positive correlation with the severity of facial erythema by AD, for example, with the erythema index. In contrast, when these markers are negative markers, the expression levels thereof indicate a negative correlation with the severity of facial erythema by AD, for example, with the erythema index. The severity of facial erythema by AD in a subject can be detected using the expression level of the positive marker or the negative marker as an indicator.

In the present embodiment, one or more markers selected from the positive markers and the negative markers in a biological specimen collected from a subject can be a target marker that is used as an indicator for the detection above. As described above, the positive markers and the negative markers are markers that are correlated with the EASI score or the POEM score associated with systemic eruption by AD, the VAS score of itching of the skin by AD, the VAS score of skin dryness by AD, or the erythema index associated with facial erythema by AD. In the present embodiment, at least one marker correlated with any of the scores and indexes above may be used as the target marker. Preferably, two or more markers that are correlated with different scores or indexes are used as a target marker. More preferably, a combination of markers that are respectively correlated with the EASI score, the POEM score, the VAS score of itching of the skin by AD, the VAS score of skin dryness by AD, and the erythema index associated with facial erythema by AD is used as the target marker. In the method of the present invention, any one of the positive markers and the negative markers may be used as the target marker, or a combination of two or more selected from the positive markers and the negative markers may be used as the target marker. Alternatively, a combination of nucleic acid markers or protein markers consisting of all the positive markers and the negative markers may be used as the target marker.

In an embodiment, the severity of AD in a subject can be detected by measuring the expression level of a target marker in the subject and comparing the measured expression level of the target marker with a preset reference value.

The reference value can be determined in advance based on a relationship between the severity of AD (the level of AD symptoms classified based on the score value associated with the severity of AD or the score value associated with the severity of AD) and the expression level of a target marker. For example, a population is divided into a plurality of groups of different severity based on the severity of AD, and a reference value for discriminating whether or not of belonging to each group can be determined with reference to the statistic (e.g., average) of the expression levels of the target marker in each group. When multiple markers are used as the target marker, it is preferable to determine a reference value for each marker. The population may be a patient group having AD, a group including healthy subjects and patients having AD, or a patient group having AD with specific severity. Alternately, the population may be created by age, generation, gender, or race according to a subject as an object for detection.

Examples of the group used for calculating reference value include a group having mild AD (mild group), a group having moderate AD (moderate group), and a group having severe AD (severe group). Alternatively, a patient group based on the classification of more detailed severity may be selected, and the reference value may be calculated for each patient group. A healthy group (group not having AD) may be included as a control.

In an example, when the severity of systemic eruption by AD (e.g., the severity of AD corresponding to the EASI score) is detected, a reference value can be calculated from two or more groups having specific AD severity grouped based on the EASI score from an AD patient group.

In another example, when the severity of systemic eruption by AD (e.g., the severity of AD corresponding to the POEM score) is detected, a reference value can be calculated from two or more groups having specific AD severity grouped based on the POEM score from an AD patient group.

In another example, when the severity of itching of the skin by AD is detected, a reference value can be calculated from two or more groups having specific AD severity grouped based on the VAS score associated with itching of the skin from an AD patient group.

In another example, when the severity of skin dryness by AD is detected, a reference value can be calculated from two or more groups having specific AD severity grouped based on the VAS score associated with skin dryness from an AD patient group.

In another example, when the severity of facial erythema by AD is detected, a reference value can be calculated from two or more groups having specific AD severity grouped based on the erythema index associated with facial erythema from an AD patient group.

When the positive marker is used, the higher the expression level thereof is, the worse the severity of AD in the subject is detected. In contrast, when the negative marker is used, the lower the expression level thereof is, the worse the severity of AD in the subject is detected.

Specific means for setting a reference value and classification of severity based on the reference value can be appropriately carried out according to common technical knowledge of those skilled in the art.

In another embodiment, a change in the severity (for example, exacerbation or remission) of AD in a subject can be detected by measuring the expression level of a target marker in the subject at different time points and comparing the measured expression levels of the target marker.

In an example, a change in the severity of systemic eruption by AD (e.g., the severity of AD corresponding to the EASI score) is detected. As the target marker, a marker for detecting the severity of systemic eruption by AD described above, for example, a marker that can detect the severity of atopic dermatitis corresponding to the EASI score is used.

In another example, a change in the severity of systemic eruption by AD (e.g., the severity of AD corresponding to the POEM score) is detected. As the target marker, a marker for detecting the severity of systemic eruption by AD described above, for example, a marker that can detect the severity of atopic dermatitis corresponding to the POEM score is used.

In another example, a change in the severity of itching of the skin by AD is detected. As the target marker, a marker for detecting the severity of itching of the skin by AD described above, for example, a marker that can detect the severity of atopic dermatitis corresponding to the VAS score of itching of the skin by AD is used.

In another example, a change in the severity of skin dryness by AD is detected. As the target marker, a maker for detecting the severity of skin dryness by AD described above, for example, a marker that can detect the severity of atopic dermatitis corresponding to the VAS score of skin dryness by AD is used.

In another example, a change in the severity of facial erythema by AD is detected. As the target marker, a marker for detecting the severity of facial erythema by AD described above, for example, a marker that can detect the severity of atopic dermatitis corresponding to the erythema index associated with facial erythema by AD is used.

When a positive marker is used, an increase in the expression level thereof over time indicates exacerbation of the severity of AD in a subject. In contrast, a decrease in the expression level over time indicates remission of the severity of AD in a subject. In an example, the expression level of a marker in the same subject at previous measurement is used as a reference value. When the measured expression level of a positive marker in a subject is higher than the reference value, the severity of AD in the subject is detected to be exacerbated. In contrast, when the measured expression level of a positive marker in a subject is lower than the reference value, the severity of AD in the subject is detected to be remitted. The severity of AD in the subject may be judged by a conventional means at the time of the previous measurement as needed. In such a case, when the measured expression level of the positive marker in a subject is higher or lower than a reference value, the severity of AD in the subject can be detected to be more severe or milder than the AD severity at the previous measurement.

When a negative marker is used, an increase in the expression level over time indicates remission of the severity of AD in a subject. In contrast, a decrease in the expression level over time indicates exacerbation of the severity of AD in a subject. In an example, the expression level of a marker in the same subject at previous measurement is used as a reference value. When the measured expression level of a negative marker in a subject is higher than the reference value, the severity of AD in the subject is detected to be remitted. In contrast, when the measured expression level of a negative marker in a subject is lower than the reference value, the severity of AD in the subject is detected to be exacerbated. The severity of AD in the subject may be judged by a conventional means at the time of the previous measurement as needed. In such a case, when the measured expression level of the negative marker in a subject is higher or lower than a reference value, the severity of AD in the subject can be detected to be milder or more severe than the AD severity at the previous measurement.

In an embodiment of the method of the present invention, when the expression level of the marker of the present invention derived from a subject with respect to a reference value is preferably 91% or less, more preferably 83% or less, and further preferably 77% or less, the expression level of the marker can be judged to be lower than the reference value, and when the expression level of the marker of the present invention with respect to a reference value is 110% or more, more preferably 120% or more, and further preferably 130% or more, the expression level of the marker can be judged to be higher than the reference value. Alternatively, a difference between the expression level of a marker derived from a subject and a reference value can be judged by, for example, whether or not the both are significantly different from each other statistically. When multiple markers are used as the target marker, the severity of AD can be detected by comparing the expression levels of the individual target markers with respective reference values, and examining whether or not the expression levels of the markers at a certain proportion, for example, 50% or more, preferably 70% or more, more preferably 90% or more, and further preferably 100%, are different from the respective reference values.

(2.3 Detection of AD severity based on prediction model)

In another embodiment of the method of the present invention, the severity of AD in a subject is detected based on a prediction model constructed using data for the expression level (hereinafter, referred to as expression profile) of the marker of the present invention derived from the subject (a marker of the present invention included in a biological specimen collected from the subject). Examples of the expression profile include data relating to the expression level such as read count of sequencing.

For example, a prediction model (e.g., discriminant) for detecting the AD severity in an arbitrary subject can be constructed by defining the expression profile of each of one or more markers (genes or expression products thereof) obtained from each person of a teacher sample population (for example, a population including multiple groups with different severity) as an explanatory variable and performing machine learning using the variable indicating the severity group to which the person in the population belongs as the objective variable. The severity of AD in a subject, specifically, the severity group to which a subject belongs can be detected using the constructed prediction model.

In the present specification, the term "feature quantity" is synonymous with "explanatory variable" in machine learning. In the present specification, the marker of which the expression profile is used as the explanatory variable (feature quantity) of machine learning may be referred to as "feature quantity marker (group)". The feature quantity marker that is a gene or transcript thereof may be referred to as a feature quantity gene.

The feature quantity marker (group) used in the present embodiment may be at least one selected from the group consisting of the genes shown in Tables 1A and 1B, and the expression products of the genes. The expression profile of the feature quantity marker may be an absolute value or a relative value or may be processed for normalization. When multiple markers are used as a feature quantity marker group, for example, multiple markers having high correlation with the severity of AD are selected from the markers of the present invention, and each of the expression profiles thereof can be used as an explanatory variable.

In an embodiment, all the genes in Table 1A or expression products thereof are combined and used as a feature quantity marker group. In another embodiment, all the genes in Table 1B or expression products thereof are combined and used as a feature quantity marker group. In another embodiment, all the genes in Tables 1A and 1B or expression products thereof are combined and used as a feature quantity marker group.

In a preferable embodiment, as a teacher sample for machine learning, used is the expression profile of a feature quantity marker (group) in a population including two or more AD patient groups with different severity (for example, two or more groups selected from a group with no symptom of AD, a mild AD group, a moderate AD group, and a severe AD group, but not limited thereto). A discriminant (prediction model) for classifying the severity of AD of subjects is constructed using the teacher sample. As the explanatory variable to be used for construction of the discriminant, the expression profile of the feature quantity marker (group) can be used. As the objective variable, for example, a variable indicating that which AD severity patient group a subject from which the feature quantity marker (group) is derived belongs to can be used. A cut-off value for discriminating AD severity can be determined based on the constructed discriminant. Subsequently, the AD severity of a subject is discriminated by measuring the expression profile of the feature quantity marker (group) derived from the subject, assigning the obtained measured value to the discriminant, and comparing the result obtained from the discriminant with the cut-off value. The cut-off value can be determined according to a known means. For example, a receiver operating characteristic (ROC) curve is determined using the constructed discriminant, and the Youden index thereof can be determined as a cut-off value.

Alternatively, when the expression profile of a marker is used for construction of a prediction model, the prediction model may be constructed after compression of the data by dimension reduction as needed. For example, multiple markers are extracted from the gene groups shown in Tables 1A and 1B or the expression products thereof. Subsequently, the expression profiles of the extracted markers are subjected to principal component analysis. A prediction model for discriminating the AD severity in a subject can be constructed by machine learning using one or more main components calculated by the principal component analysis as the explanatory variable and a variable indicating that which severity group (for example, a mild group or a severe group) the subject from which the explanatory variable is derived belongs to as the objective variable.

As the algorithm in construction of a prediction model, for example, a known algorithm that is used in machine learning can be used. Examples of the machine learning algorithm include, but not limited to, a linear regression model (Linear model), Lasso regression (Lasso), random forest, neural network (Neural net), linear kernel support vector machine (SVM (linear)), rbf kernel support vector machine (SVM (rbf)), regularized linear discriminant analysis, and regularized logistic regression.

A predicted value is calculated by inputting data for verification into the constructed prediction model. A model of which the predicted values best fit the measured values, for example, a model having the highest accuracy of the predicted values to measured values, can be selected as the optimum model. Alternatively, the recall, the precision, and the harmonic mean thereof, F value, are calculated from the predicted values and the measured values, and a model having the largest F value can be selected as the optimum model. The AD severity in a subject can be detected by inputting the expression profile of the feature quantity marker (group) actually measured for the subject into the constructed prediction model.

(3. Kit for detecting AD severity)

In a further aspect, the present invention provides a kit for detecting the severity of AD in a subject according to the method of the present invention described in the paragraph 2. above. In an embodiment, the kit of the present invention includes a reagent or tool for measuring the expression level of the marker of the present invention described above. For example, the kit of the present invention can include reagents for amplifying or quantitatively measuring the nucleic acid marker of the present invention (for example, a reverse transcriptase, a reagent for PCR, a primer, a probe, and an adaptor sequence for sequencing) or reagents for quantitatively measuring the protein marker of the present invention (for example, a reagent and an antibody for immunoassay). Preferably, the kit of the present invention includes an oligonucleotide that specifically hybridizes with the nucleic acid marker of the present invention (for example, a primer or probe for PCR) or an antibody that recognizes the protein marker of the present invention. Preferably, the kit of the present invention includes an indicator or guidance for assessing the expression level of the marker of the present invention. For example, the kit of the present invention can include, for example, guidance for describing AD symptoms (for example, eruption, skin itching, skin dryness, and facial erythema) associated with each marker, guidance for describing a relationship between an increase or decrease in the expression level of each marker and AD severity, guidance for describing a reference value of the expression level of each marker for detecting the severity of AD, or guidance for a discriminant based on the prediction model and a feature quantity marker to be input thereinto. The kit of the present invention may further include a biological sampling device (for example, the above-mentioned SSL-absorptive material or SSL-adhesive material), a reagent for extracting the marker of the present invention from a biological specimen (for example, a reagent for nucleic acid purification), and a preservative and a storage container for the sampling device after collection of a biological specimen.

As exemplary embodiments of the present invention, the following materials, production methods, uses, methods, and so on are further disclosed herein. However, the present invention is not limited to these embodiments.

[1] A marker for detecting severity of atopic dermatitis, comprising at least one selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes.

[2] The marker according to [1], wherein the severity of atopic dermatitis is preferably severity of systemic eruption by atopic dermatitis, severity of itching of skin by atopic dermatitis, severity of dryness of skin by atopic dermatitis, or severity of facial erythema by atopic dermatitis.

[3] The marker according to [1] or [2], wherein the marker is preferably a marker for detecting the severity of systemic eruption by atopic dermatitis and comprises at least one selected from the group consisting of following genes: CIZ1, ADAM15, SETD1B, and TWF1, and expression products of the genes.

[4] The marker according to [3], wherein the marker is preferably a marker for detecting severity of atopic dermatitis corresponding to Eczema Area and Severity Index.

[5] The marker according to [1] or [2], wherein the marker is preferably a marker for detecting the severity of systemic eruption by atopic dermatitis and comprise at least one selected from the group consisting of following genes: LYNX1 and PSME2, and expression products of the genes.

[6] The marker according to [5], wherein the marker is preferably a marker for detecting severity of atopic dermatitis corresponding to Patient Oriented Eczema Measure.

[7] The marker according to [1] or [2], wherein the marker is preferably a marker for detecting the severity of itching of the skin by atopic dermatitis and comprises at least one selected from the group consisting of following genes: ALPK1, ATG16L2, CSNK1D, GSK3A, LSM10, SASH3, and VSIR, and expression products of the genes.

[8] The marker according to [7], wherein the marker is preferably a marker for detecting severity of atopic dermatitis corresponding to Visual Analog Scaling score of itching of skin by atopic dermatitis.

[9] The marker according to [1] or [2], wherein the marker is preferably a marker for detecting the severity of skin dryness by atopic dermatitis and comprise at least one selected from the group consisting of following genes: TSC22D3, PLXNC1, and SLC12A6, and expression products of the genes.

[10] The marker according to [9], wherein the marker is preferably a marker for detecting severity of atopic dermatitis corresponding to Visual Analog Scaling score of skin dryness by atopic dermatitis.

[11] The marker according to [1] or [2], wherein the marker is preferably a marker for detecting the severity of facial erythema by atopic dermatitis and comprise at least one selected from the group consisting of following genes: ODC1, AGR2, FASN, APOD, ITPKB, and PDK4 gene, and expression products of the genes.

[12] The marker according to [11], wherein the marker is preferably a marker for detecting severity of atopic dermatitis corresponding to erythema index associated with facial erythema by atopic dermatitis.

[13] The marker according to any one of [1] to [12], wherein the marker is preferably a nucleic acid marker.

[14] The marker according to [13], wherein the nucleic acid is preferably mRNA collected from skin surface lipids.

[15] A method for acquiring data for detecting severity of atopic dermatitis in a subject, the method comprising measuring an expression level of the marker according to any one of [1] to [14] in a subject.

[16] A method for detecting severity of atopic dermatitis in a subject, the method comprising measuring an expression level of the marker according to any one of [1] to [14] in a subject.

[17] The method according to [16], preferably further comprising detecting severity of atopic dermatitis in the subject based on the expression level of the marker.

[18] The method according to any one of [15] to [17], wherein the marker is preferably the marker according to [3], and the severity is severity of systemic eruption by atopic dermatitis.

[19] The method according to [18], wherein the severity is preferably severity of atopic dermatitis corresponding to Eczema Area and Severity Index.

[20] The method according to any one of [15] to [17], wherein the marker is preferably the marker according to [5], and the severity is severity of systemic eruption by atopic dermatitis.

[21] The method according to [20], wherein the severity is preferably severity of atopic dermatitis corresponding to Patient Oriented Eczema Measure.

[22] The method according to any one of [15] to [17], wherein the marker is preferably the marker according to [7], and the severity is severity of itching of the skin by atopic dermatitis.

[23] The method according to [22], wherein the severity is preferably severity of atopic dermatitis corresponding to Visual Analog Scaling score of itching of skin by atopic dermatitis.

[24] The method according to any one of [15] to [17], wherein the marker is preferably the marker according to [9], and the severity is severity of dryness of skin by atopic dermatitis.

[25] The method according to [24], wherein the severity is preferably severity of atopic dermatitis corresponding to Visual Analog Scaling score of skin dryness by atopic dermatitis.

[26] The method according to any one of [15] to [17], wherein the marker is preferably the marker according to [11], and the severity is severity of facial erythema by atopic dermatitis.

[27] The method according to [26], wherein the severity is preferably severity of atopic dermatitis corresponding to erythema index associated with facial erythema by atopic dermatitis.

[28] The method according to any one of [16] to [27], wherein the marker is preferably at least one selected from the group consisting of the genes shown in Table 1A above and expression products of the genes, and the higher the expression level of the marker is, the worse the severity of atopic dermatitis in the subject is detected.

[29] The method according to any one of [16] to [27], wherein the marker is preferably at least one selected from the group consisting of the genes shown in Table 1B above and expression products of the genes, and the lower the expression level of the marker is, the worse the severity of atopic dermatitis in the subject is detected.

[30] The method according to any one of [15] to [27], preferably comprising measuring the expression level of the marker in the subject at different time points.

[31] The method according to [30], wherein

the marker is preferably at least one selected from the group consisting of the genes shown in Table 1A above and expression products of the genes, and

when the measured expression level of the marker in the subject is higher than the expression level in the subject at previous measurement, the severity of atopic dermatitis in the subject is detected to be exacerbated, or when the measured expression level of the marker in the subject is lower than the expression level in the subject at previous measurement, the severity of atopic dermatitis in the subject is detected to be remitted.

[32] The method according to [30], wherein

the marker is preferably at least one selected from the group consisting of the genes shown in Table 1B above and expression products of the genes, and

when the measured expression level of the marker in the subject is lower than the expression level in the subject at previous measurement, the severity of atopic dermatitis in the subject is detected to be exacerbated, or when the measured expression level of the marker in the subject is higher than the expression level in the subject at previous measurement, the severity of atopic dermatitis in the subject is detected to be remitted.

[33] A kit for detecting severity of atopic dermatitis for use in the method according to any one of [15] to [32,] the kit comprising an oligonucleotide that specifically hybridizes with a nucleic acid as the marker according to any one of [1] to [12] or an antibody that recognizes a protein as the marker according to any one of [1] to [12].

[34] Use of at least one selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes as a marker for detecting severity of atopic dermatitis.

[35] Use of at least one selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes in production of a marker for detecting severity of atopic dermatitis.

[36] The use according to [34] or [35], wherein the severity of atopic dermatitis is severity of systemic eruption by atopic dermatitis, severity of itching of skin by atopic dermatitis, severity of dryness of skin by atopic dermatitis, or severity of facial erythema by atopic dermatitis.

[37] The use according to any one of [34] to [36], wherein the marker is preferably a marker for detecting the severity of systemic eruption by atopic dermatitis, and the marker comprises at least one selected from the group consisting of following genes: CIZ1, ADAM15, SETD1B, and TWF1, and expression products of the genes.

[38] The use according to [37], wherein the marker is preferably a marker for detecting severity of atopic dermatitis corresponding to Eczema Area and Severity Index.

[39] The use according to any one of [34] to [36], wherein the marker is preferably a marker for detecting the severity of systemic eruption by atopic dermatitis, and the marker comprises at least one selected from the group consisting of following genes: LYNX1 and PSME2, and expression products of the genes.

[40] The use according to [39], wherein the marker is preferably a marker for detecting severity of atopic dermatitis corresponding to Patient Oriented Eczema Measure.

[41] The use according to any one of [34] to [36], wherein the marker is preferably a marker for detecting the severity of itching of the skin by atopic dermatitis, and the marker comprises at least one selected from the group consisting of following genes: ALPK1, ATG16L2, CSNK1D, GSK3A, LSM10, SASH3, and VSIR, and expression products of the genes.

[42] The use according to [41], wherein the marker is preferably a marker for detecting severity of atopic dermatitis corresponding to Visual Analog Scaling score of itching of skin by atopic dermatitis.

[43] The use according to any one of [34] to [36], wherein the marker is preferably a marker for detecting the severity of skin dryness by atopic dermatitis, and the marker comprises at least one selected from the group consisting of following genes: TSC22D3, PLXNC1, and SLC12A6, and expression products of the genes.

[44] The use according to [43], wherein the marker is preferably a marker for detecting severity of atopic dermatitis corresponding to Visual Analog Scaling score of skin dryness by atopic dermatitis.

[45] The use according to any one of [34] to [36], wherein the marker is preferably a marker for detecting the severity of facial erythema by atopic dermatitis, and the marker comprises at least one selected from the group consisting of following genes: ODC1, AGR2, FASN, APOD, ITPKB, and PDK4 gene, and expression products of the genes.

[46] The use according to [45], wherein the marker is preferably a marker for detecting severity of atopic dermatitis corresponding to erythema index associated with facial erythema by atopic dermatitis.

[47] The use according to any one of [34] to [46], wherein the marker is preferably a nucleic acid marker.

[48] The use according to [47], wherein the nucleic acid is preferably mRNA collected from skin surface lipids.

Examples

The present invention will now be described in more detail based on examples, but is not limited thereto.

Example 1: Search for marker for detecting severity of atopic dermatitis using RNA derived from SSL

1) Acquisition of score associated with severity of atopic dermatitis patient and SSL collection

Subjects were 18 adults (23- to 57-year old males) having atopic dermatitis (AD). The subjects were AD patients who were diagnosed by a dermatologist as having mild or moderate atopic dermatitis at the first measurement. The subjects visited four times every 14 days and were subjected to acquisition of a score associated with the severity of AD and collection of SSL. Hereinafter, the obtained scores associated with the severity of AD and the collected SSL are referred to as 1st, 2nd, 3rd, and 4th scores and SSL samples, respectively, based on the order from the first visit. As the score associated with the severity of AD, the systemic EASI score (Hanifin, et al., Exp. Dermatol., 10, 2001, scoring each symptom from 0 to 72 based on systemic eruption) by the dermatologist, the systemic POEM score (Charman, et al., Arch. Dermatol., 140, 2004, scoring each symptom from 0 to 28 based on systemic eruption) by the subject himself, the VAS score of itching of the systemic skin (scoring the degree of itching from 0 to 100) and the VAS score of dryness of the systemic skin (scoring the degree of dryness from 0 to 100), and the facial erythema index based on the face image by a hyper spectral imaging apparatus (hyper spectral camera NH-7, EBA Japan Co., Ltd.) (see JP-A-2018-23756 and Dawson, et al., Phys. Med. Biol., 25, 1980) were each used. In calculation of the facial erythema index, the erythema index was calculated for each pixel on a front face image by the hyper spectral imaging apparatus according to the equation (1) below. An arbitrary region of interest (ROI) was determined in each of the areas corresponding to forehead, upper parts of both eyes, and both cheeks in an image, and the average of erythema indexes at five ROIs was used as the erythema index of the face.

$$\text{Erythema index} = 100 \{A_{560} + 1.5(A_{543} + A_{576}) - 2(A_{510} + A_{610})\} \quad \dots$$

$$(1)$$

Here, $A_\lambda = \log_{10}(1/R_\lambda)$

$A_\lambda$; Apparent absorbance at wavelength $\lambda$
$R_\lambda$; Reflectance at wavelength $\lambda$

Sebum was collected from the whole face of each subject using an oil blotting film (5 × 8 cm, made of polypropylene, 3M Company). The oil blotting film was transferred into a vial and was stored at -80°C for about one month before use for RNA extraction.

2) RNA preparation and sequencing

The oil blotting film of the above 1) was cut into an appropriate size, and RNA was transferred into a water layer using QIAzol Lysis Reagent (Qiagen N.V.) according to the attached protocol. The RNA was extracted from the water layer using a commercially available RNA extraction kit using a spin column for RNA extraction according to the attached protocol. The extracted RNA was reverse-transcribed using SuperScript VILO cDNA Synthesis kit (Life Technologies Japan Ltd.) at 42°C for 90 minutes to synthesize cDNA. As the primer for the reverse transcription reaction, the random primer attached to the kit was used. A library containing DNA derived from 20,802 genes was prepared from the resulting cDNA by multiplex PCR. The multiplex PCR was performed using Ion AmpliSeq Transcriptome Human Gene Expression Kit (Life Technologies Japan Ltd.) under conditions of [99°C for 2 min → 20 cycles of (99°C for 15 sec -> 62°C for 16 min) → 4°C for hold]. The obtained PCR products were purified with Ampure XP (Beckman Coulter, Inc.), and then buffer reconstitution, digestion of primer sequences, adaptor ligation and purification, and amplification were performed to prepare the library. The prepared library was loaded on Ion 540 Chip, and sequencing was performed using Ion S5/XL system (Life Technologies Japan Ltd.). Each of the read sequences obtained by sequencing was genetically mapped using a reference sequence, hg19 AmpliSeq Transcriptome ERCC v1, of the human genome to determine the gene from which each read sequence was derived.

3) Data used

The read count of each read by sequencing of RNA derived from SSL of each subject measured in the above 2) was used as data of the expression level of each RNA. A gene whose amplified region in the sequencing spanned at least two or more exons was used as an analysis target gene. In order to normalize the total read count for the difference between samples, the read count of the analysis target gene was converted to the RPM (reads per million mapped reads) value. Among them, 4,845 genes with read counts of 20 or more in 90% or more samples were used in the following analysis. Furthermore, in order to approximate the RPM values to a normal distribution, the RPM value was converted to the base-2 logarithmic value of the RPM value plus an integer 1 ($Log_2$(RPM+1) value). According to the procedure above, expression level data ($Log_z$(RPM+1) values) of 4,845 genes were each produced for the 1st, 2nd, 3rd, and 4th SSL samples of the 18 subjects. These data are each referred to as 1st, 2nd, 3rd, and 4th gene expression level data based on the order from the first visit.

4) Data analysis

i) Search for gene correlated with EASI score

Based on the 1st EASI scores of the 18 AD patients acquired in the above 1) and the 1st gene expression level data ($Log_2$(RPM+1) values) of 4,845 genes of the 18 AD patients calculated in the above 3), the Spearman's correlation coefficient Rs between the EAST score and the expression level of each gene was calculated. Similarly, Rs was calculated between the 2nd to 4th EASI scores and the gene expression levels, respectively. The Rs each calculated was referred to as 1st to 4th Rs for each gene.

Regarding each gene, the number of times the p value (p_val) was below 0.1 in the 1st to 4th Rs (this number of times was defined as A value) and the number of times the p value was below 0.05 in the 1st to 4th Rs (this number of times was defined as B value) were examined. Four genes, CIZ1, ADAM15, STED1B, and TWF1, shown in Table 2 had an A value of 4 or a B value of 3 or more, and the correlation with the EAST score was high. There have been no reports to date suggesting that these four genes are related to atopic dermatitis. Accordingly, it was judged that these genes can be new markers for detecting the severity of atopic dermatitis.

[Table 2]

| Gene Symbol | Rs (1st) | p_val (1st) | Rs (2nd) | p_val (2nd) | Rs (3rd) | p_val (3rd) | Rs (4th) | p_val (4th) | A value | B value |
|---|---|---|---|---|---|---|---|---|---|---|
| CIZ1 | 0.4159 | 0.0874 | 0.5046 | 0.0346 | 0.5978 | 0.0088 | 0.4318 | 0.0735 | 4 | 2 |
| ADAM15 | 0.4799 | 0.0457 | 0.3375 | 0.1709 | 0.5627 | 0.0150 | 0.5310 | 0.0234 | 3 | 3 |
| SETD1B | 0.00722 | 0.9803 | 0.5872 | 0.0119 | 0.4915 | 0.0383 | 0.5579 | 0.0161 | 3 | 3 |
| TWF1 | 0.2962 | 0.2320 | 0.5335 | 0.0244 | 0.6164 | 0.0064 | 0.5702 | 0.0135 | 3 | 3 |

ii) Search for gene correlated with POEM score

Based on the 1st POEM scores of the 18 AD patients acquired in the above 1) and the 1st gene expression level data ($Log_2$(RPM+1) values) of 4,845 genes of the 18 AD patients calculated in the above 3), the Spearman's correlation coefficient Rs between the POEM score and the expression level of each gene was calculated. Similarly, the Rs was calculated between the 2nd to 4th POEM scores and gene expression levels, respectively. The Rs each calculated was referred to as 1st to 4th Rs for each gene.

Regarding each gene, the number of times the p value (p_val) was below 0.1 in the 1st to 4th Rs (this number of times was defined as A value) and the number of times the p value was below 0.05 in the 1st to 4th Rs (this number of times was defined as B value) were examined. Two genes, LYNX1 and PSME, shown in Table 3 had an A value of 4 or a B value of 3 or more, and the correlation with the POEM score was high. There have been no reports to date suggesting that these two genes are related to atopic dermatitis. Accordingly, it was judged that these genes can be new markers for detecting the severity of atopic dermatitis.

[Table 3]

| Gene Symbol | Rs (1st) | p_val (1st) | Rs (2nd) | p_val (2nd) | Rs (3rd) | p_val (3rd) | Rs (4th) | p_val (4th) | A value | B value |
|---|---|---|---|---|---|---|---|---|---|---|
| LYNX1 | 0.1634 | 0.5171 | 0.4834 | 0.0421 | 0.6404 | 0.00419 | 0.4834 | 0.04211 | 3 | 3 |
| PSME2 | 0.3258 | 0.1871 | 0.4761 | 0.0458 | 0.5606 | 0.0155 | 0.5457 | 0.0192 | 3 | 3 |

iii) Search for gene correlated with VAS score of skin itching

Based on the 1st VAS score of skin itching of the 18 AD patients acquired in the above 1) and the 1st gene expression level data (Logz(RPM+1) values) of 4,845 genes of the 18 AD patients calculated in the above 3), the Spearman's correlation coefficient Rs between the VAS score and the expression level of each gene was calculated. Similarly, the Rs between the 2nd to 4th VAS scores and gene expression levels was respectively calculated. The Rs each calculated was referred to as 1st to 4th Rs for each gene, respectively.

Regarding each gene, the number of times the p value (p_val) was below 0.1 in the 1st to 4th Rs (this number of times was defined as A value) and the number of times the p value was below 0.05 in the 1st to 4th Rs (this number of times was defined as B value) were examined. Seven genes, ALPK1, ATG16L2, CSNK1D, GSK3A, LSM10, SASH3, and VSIR, shown in Table 4 had an A value of 4 or a B value of 3 or more, and the correlation with itching was high. There have been no reports to date suggesting that these seven genes are related to atopic dermatitis. Accordingly, it was judged that these genes can be new markers for detecting the severity of atopic dermatitis.

[Table 4]

| Gene Symbol | Rs (1st) | p_val (1st) | Rs (2nd) | p_val (2nd) | Rs (3rd) | p_val (3rd) | Rs (4th) | p_val (4th) | A value | B value |
|---|---|---|---|---|---|---|---|---|---|---|
| ALPK1 | -0.0703 | 0.7817 | -0.4977 | 0.0356 | -0.5700 | 0.0135 | -0.5323 | 0.0230 | 3 | 3 |
| ATG16L2 | 0.0062 | 0.9805 | -0.5442 | 0.0195 | -0.5503 | 0.0180 | -0.5530 | 0.0173 | 3 | 3 |
| CSNK1D | -0.3473 | 0.1579 | -0.5587 | 0.0159 | -0.4812 | 0.0432 | -0.5850 | 0.0108 | 3 | 3 |
| GSK3A | 0.1736 | 0.4908 | -0.5060 | 0.0322 | -0.4843 | 0.0417 | -0.4765 | 0.0456 | 3 | 3 |
| LSM10 | -0.1860 | 0.4598 | -0.5008 | 0.0343 | -0.5958 | 0.0091 | -0.5354 | 0.0220 | 3 | 3 |
| SASH3 | -0.0610 | 0.8100 | -0.5908 | 0.00983 | -0.4750 | 0.0464 | -0.4972 | 0.0358 | 3 | 3 |
| VSIR | 0.0579 | 0.8195 | -0.7087 | 0.00099 | -0.5348 | 0.0222 | -0.4889 | 0.0395 | 3 | 3 |

iv) Search for gene correlated with VAS score of skin dryness

Based on the 1st VAS score of skin dryness of the 18 AD patients acquired in the above 1) and the 1st gene expression level data ($Log_2$(RPM+1) values) of 4,845 genes of the 18 AD patients calculated in the above 3), the Spearman's correlation coefficient Rs between the VAS score and the expression level of each gene was calculated. Similarly, the R between the 2nd to 4th VAS scores and gene expression levels were respectively calculated. The Rs each calculated referred to as 1st to 4th Rs for each gene, respectively.

Regarding each gene, the number of times the p value (p_val) was below 0.1 in the 1st to 4th Rs (this number of times was defined as A value) and the number of times the p value was below 0.05 in the 1st to 4th Rs (this number of times was defined as B value) were examined. Three genes, TSC22D3, PLXNC1, and SLC12A6, shown in Table 5 had an A value of 4 or a B value of 3 or more, and the correlation with dryness was high. There have been no reports to date suggesting that these three genes are related to atopic dermatitis. Accordingly, it was judged that these genes can be new markers for detecting the severity of atopic dermatitis.

[Table 5]

| Gene Symbol | Rs (1st) | p_val (1st) | Rs (2nd) | p_val (2nd) | Rs (3rd) | p_val (3rd) | Rs (4th) | p_val (4th) | A value | B value |
|---|---|---|---|---|---|---|---|---|---|---|
| TSC22D3 | -0.4041 | 0.0962 | -0.4397 | 0.0678 | -0.40305 | 0.0980 | -0.4943 | 0.0389 | 4 | 1 |
| PLXNC1 | -0.0879 | 0.7289 | -0.4956 | 0.0365 | -0.4861 | 0.0427 | -0.4757 | 0.0478 | 3 | 3 |
| SLC12A6 | -0.1478 | 0.5584 | -0.6291 | 0.00516 | -0.5253 | 0.0270 | -0.4861 | 0.0427 | 3 | 3 |

v) Search for gene correlated with facial erythema index

Based on the 1st facial erythema index of the 18 AD patients acquired in the above 1) and the 1st gene expression level data ($Log_2$(RPM+1) values) of 4,845 genes of the 18 AD patients calculated in the above 3), the Spearman's correlation coefficient Rs between the facial erythema index and the expression level of each gene was calculated. Similarly, the Rs between the 2nd to 4th facial erythema indexes and gene expression levels were respectively calculated. The Rs each calculated referred to as 1st to 4th Rs for each gene, respectively.

Regarding each gene, the number of times the p value (p_val) was below 0.1 in the 1st to 4th Rs (this number of times was defined as A value) and the number of times the p value was below 0.05 in the 1st to 4th Rs (this number of times was defined as B value) were examined. ODC1 shown in Table 6A had a positive correlation with the facial erythema index, and five genes, AGR2, FASN, APOD, ITPKB, and PDK4, shown in Table 6B had a negative correlation with the facial erythema index. These six genes in total had an A value of 4 or a B value of 3 or more, and the correlation with facial erythema was high. There have been no reports to date suggesting that these six genes are related to atopic dermatitis. Accordingly, it was judged that these genes can be new markers for detecting the severity of atopic dermatitis.

[Table 6]

A

| Gene Symbol | Rs (1st) | p_val (1st) | Rs (2nd) | p_val (2nd) | Rs (3rd) | p_val (3rd) | Rs (4th) | p_val (4th) | A value | B value |
|---|---|---|---|---|---|---|---|---|---|---|
| ODC1 | 0.5748 | 0.0141 | 0.6883 | 0.00214 | 0.5459 | 0.0208 | 0.0939 | 0.7109 | 3 | 3 |

B

| Gene Symbol | Rs (1st) | p_val (1st) | Rs (2nd) | p_val (2nd) | Rs (3rd) | p_val (3rd) | Rs (4th) | p_val (4th) | A value | B value |
|---|---|---|---|---|---|---|---|---|---|---|
| AGR2 | -0.5893 | 0.0115 | -0.4345 | 0.0731 | -0.5088 | 0.0329 | -0.4469 | 0.0647 | 4 | 2 |
| FASN | -0.4572 | 0.0582 | -0.4675 | 0.0522 | -0.4530 | 0.0607 | -0.4613 | 0.0557 | 4 | 0 |
| APOD | -0.5666 | 0.0158 | -0.6305 | 0.00609 | -0.5501 | 0.0198 | -0.3354 | 0.1736 | 3 | 3 |
| ITPKB | 0.2714 | 0.2749 | -0.4840 | 0.0437 | -0.4985 | 0.0371 | -0.6037 | 0.00929 | 3 | 3 |
| PDK4 | -0.5356 | 0.0238 | -0.4778 | 0.0467 | -0.5026 | 0.0354 | -0.0960 | 0.7048 | 3 | 3 |

**Claims**

1.  A method for detecting severity of atopic dermatitis in a subject, the method comprising:

    measuring an expression level of a marker for detecting severity of atopic dermatitis in a subject, wherein the marker for detecting severity of atopic dermatitis is at least one selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes.

2.  The method according to claim 1, further comprising detecting severity of atopic dermatitis in the subject based on the expression level of the marker.

3.  The method according to claim 1 or 2, wherein the marker is at least one selected from the group consisting of following genes: CIZ1, ADAM15, SETD1B, and TWF1, and expression products of the genes, and the severity is severity of systemic eruption by atopic dermatitis.

4.  The method according to claim 3, wherein the severity is severity of atopic dermatitis corresponding to Eczema Area and Severity Index.

5.  The method according to claim 1 or 2, wherein the marker is at least one selected from the group consisting of following genes: LYNX1 and PSME2, and expression products of the genes, and the severity is severity of systemic eruption by atopic dermatitis.

6.  The method according to claim 5, wherein the severity is severity of atopic dermatitis corresponding to Patient Oriented Eczema Measure.

7.  The method according to claim 1 or 2, wherein the marker is at least one selected from the group consisting of following genes: ALPK1, ATG16L2, CSNK1D, GSK3A, LSM10, SASH3, and VSIR, and expression products of the genes, and the severity is severity of itching of skin by atopic dermatitis.

8.  The method according to claim 7, wherein the severity is severity of atopic dermatitis corresponding to Visual Analog Scaling score of itching of skin by atopic dermatitis.

9.  The method according to claim 1 or 2, wherein the marker is at least one selected from the group consisting of following genes: TSC22D3, PLXNC1, and SLC12A6, and expression products of the genes, and the severity is severity of dryness of skin by atopic dermatitis.

10. The method according to claim 9, wherein the severity is severity of atopic dermatitis corresponding to Visual Analog Scaling score of skin dryness by atopic dermatitis.

11. The method according to claim 1 or 2, wherein the marker is at least one selected from the group consisting of following genes: ODC1, AGR2, FASN, APOD, ITPKB, and PDK4 gene, and expression products of the genes, and the severity is severity of facial erythema by atopic dermatitis.

12. The method according to claim 11, wherein the severity is severity of atopic dermatitis corresponding to erythema index associated with facial erythema by atopic dermatitis.

13. The method according to any one of claims 1 to 12, wherein the marker is at least one selected from the group consisting of genes shown in Table 1 and expression products of the genes, and the higher the expression level of the marker is, the worse the severity of atopic dermatitis in the subject is detected.

[Table 1]

| Gene Symbol |
| --- |
| ADAM 15 |

(continued)

| Gene Symbol |
| --- |
| CIZ1 |
| LYNX1 |
| ODC1 |
| PSME2 |
| SETD1B |
| TWF1 |

14. The method according to any one of claims 1 to 12, wherein the marker is at least one selected from the group consisting of genes shown in Table 2 and expression products of the genes, and the lower the expression level of the marker is, the worse the severity of atopic dermatitis in the subject is detected.

[Table 2]

| Gene Symbol | Gene Symbol |
| --- | --- |
| AGR2 | LSM10 |
| ALPK1 | PDK4 |
| APOD | PLXNC1 |
| ATG16L2 | SASH3 |
| CSNK1D | SLC12A6 |
| FASN | TSC22D3 |
| GSK3A | VSIR |
| ITPKB | |

15. The method according to any one of claims 1 to 12, comprising measuring the expression level of the marker in the subject at different time points.

16. The method according to claim 15, wherein

the marker is at least one selected from the group consisting of genes shown in Table 3 and expression products of the genes, and
when the measured expression level of the marker in the subject is higher than the expression level in the subject at previous measurement, the severity of atopic dermatitis in the subject is detected to be exacerbated, or when the measured expression level of the marker in the subject is lower than the expression level in the subject at previous measurement, the severity of atopic dermatitis in the subject is detected to be remitted.

[Table 3]

| Gene Symbol |
| --- |
| ADAM15 |
| CIZ1 |
| LYNX1 |
| ODC1 |
| PSME2 |
| SETD1B |

(continued)

| Gene Symbol |
| --- |
| TWF1 |

17. The method according to claim 15, wherein

the marker is at least one selected from the group consisting of genes shown in Table 4 and expression products of the genes, and

when the measured expression level of the marker in the subject is lower than the expression level in the subject at previous measurement, the severity of atopic dermatitis in the subject is detected to be exacerbated, or when the measured expression level of the marker in the subject is higher than the expression level in the subject at previous measurement, the severity of atopic dermatitis in the subject is detected to be remitted.

[Table 4]

| Gene Symbol | Gene Symbol |
| --- | --- |
| AGR2 | LSM10 |
| ALPK1 | PDK4 |
| APOD | PLXNC1 |
| ATG16L2 | SASH3 |
| CSNK1D | SLC12A6 |
| FASN | TSC22D3 |
| GSK3A | VSIR |
| ITPKB | |

18. Use of at least one selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes as a marker for detecting severity of atopic dermatitis.

19. Use of at least one selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and expression products of the genes in production of a marker for detecting severity of atopic dermatitis.

20. The use according to claim 18 or 19, wherein the severity of atopic dermatitis is severity of systemic eruption by atopic dermatitis, severity of itching of skin by atopic dermatitis, severity of dryness of skin by atopic dermatitis, or severity of facial erythema by atopic dermatitis.

21. The use according to any one of claims 18 to 20, wherein the marker is a marker for detecting severity of systemic eruption by atopic dermatitis, and the marker comprises at least one selected from the group consisting of following genes: CIZ1, ADAM15, SETD1B, and TWF1, and expression products of the genes.

22. The use according to claim 21, wherein the marker is a marker for detecting severity of atopic dermatitis corresponding to Eczema Area and Severity Index.

23. The use according to any one of claims 18 to 20, wherein the marker is a marker for detecting severity of systemic eruption by atopic dermatitis, and the marker comprises at least one selected from the group consisting of following genes: LYNX1 and PSME2, and expression products of the genes.

24. The use according to claim 23, wherein the marker is a marker for detecting severity of atopic dermatitis corresponding to Patient Oriented Eczema Measure.

25. The use according to any one of claims 18 to 20, wherein the marker is a marker for detecting severity of itching of skin by atopic dermatitis, and the marker comprises at least one selected from the group consisting of following genes: ALPK1, ATG16L2, CSNK1D, GSK3A, LSM10, SASH3, and VSIR, and expression products of the genes.

26. The use according to claim 25, wherein the marker is a marker for detecting severity of atopic dermatitis corresponding to Visual Analog Scaling score of itching of skin by atopic dermatitis.

27. The use according to any one of claims 18 to 20, wherein the marker is a marker for detecting severity of skin dryness by atopic dermatitis, and the marker comprises at least one selected from the group consisting of following genes: TSC22D3, PLXNC1, and SLC12A6, and expression products of the genes.

28. The use according to claim 27, wherein the marker is a marker for detecting severity of atopic dermatitis corresponding to Visual Analog Scaling score of skin dryness by atopic dermatitis.

29. The use according to any one of claims 18 to 20, wherein the marker is a marker for detecting severity of facial erythema by atopic dermatitis, and the marker comprises at least one selected from the group consisting of following genes: ODC1, AGR2, FASN, APOD, ITPKB, and PDK4 gene, and expression products of the genes.

30. The use according to claim 29, wherein the marker is a marker for detecting severity of atopic dermatitis corresponding to erythema index associated with facial erythema by atopic dermatitis.

31. The use according to any one of claims 18 to 30, wherein the marker is a nucleic acid marker.

32. The use according to claim 31, wherein the nucleic acid is mRNA collected from skin surface lipids.

33. A kit for detecting severity of atopic dermatitis for use in the method according to any one of claims 1 to 17, the kit comprising:
    an oligonucleotide that specifically hybridizes with at least one nucleic acid selected from the group consisting of following genes: ADAM15, AGR2, ALPK1, APOD, ATG16L2, CIZ1, CSNK1D, FASN, GSK3A, ITPKB, LSM10, LYNX1, ODC1, PDK4, PLXNC1, PSME2, SASH3, SETD1B, SLC12A6, TSC22D3, TWF1, and VSIR, and transcription products of the genes, or an antibody that recognizes at least one protein selected from the group consisting of translation products of the genes.

## INTERNATIONAL SEARCH REPORT

| International application No. |
| --- |
| **PCT/JP2021/043714** |

### A. CLASSIFICATION OF SUBJECT MATTER

*C12N 15/12*(2006.01)i; *C12N 15/11*(2006.01)i; *C12Q 1/6813*(2018.01)i; *C12Q 1/6883*(2018.01)i; *G01N 33/50*(2006.01)i; *G01N 33/53*(2006.01)i

FI: C12N15/12; G01N33/53 M; G01N33/50 Q; C12N15/11 Z; C12Q1/6813 Z; C12Q1/6883 Z ZNA

According to International Patent Classification (IPC) or to both national classification and IPC

### B. FIELDS SEARCHED

Minimum documentation searched (classification system followed by classification symbols)

C12N; C12Q; G01N

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-1818672 B1 (GACHON UNIVERSITY OF INDUSTRY-ACADEMIC COOPERATION FOUNDATION) 16 January 2018 (2018-01-16) claims, paragraphs [0001]-[0020], [0035]-[0038], [0111]-[0135] | 1-2, 14, 18-20, 31, 33 |
| Y | claims, paragraphs [0001]-[0020], [0035]-[0038], [0111]-[0135] | 1-33 |
| Y | JP 2020-074769 A (KAO CORP) 21 May 2020 (2020-05-21) claims, paragraphs [0013], [0030]-[0048], test examples 3, 6, tables 7-1 to 7-24 | 1-33 |
| Y | JP 2020-520938 A (DS BIOPHARMA LIMITED) 16 July 2020 (2020-07-16) paragraph [0094] | 1-33 |
| Y | JP 2017-198632 A (SHISEIDO CO LTD) 02 November 2017 (2017-11-02) paragraphs [0004]-[0005] | 1-33 |

☑ Further documents are listed in the continuation of Box C.  ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **18 January 2022** | **01 February 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

# EP 4 253 547 A1

**INTERNATIONAL SEARCH REPORT**

International application No.

**PCT/JP2021/043714**

| C. | DOCUMENTS CONSIDERED TO BE RELEVANT | |
| --- | --- | --- |
| Category* | Citation of document, with indication, where appropriate, of the relevant passages | Relevant to claim No. |
| Y | 川崎　洋、外3名, アトピー性皮膚炎のリバーストランスレーショナルリサーチ, 実験医学, January 2017, vol. 35, no. 1, pp. 33-39<br>　　p. 33, right column, line 4 to p. 36, left column, line 4, p. 37, left column, line 5 from the bottom to p. 38, line 1, fig. 3-4, (KAWASAKI, Hiroshi et al. Experimental Medicine.), non-official translation (Reverse translational research on atopic dermatitis) | 1-33 |
| Y | WO 2018/161062 A1 (CHILDREN'S HOSPITAL MEDICAL CENTER) 07 September 2018 (2018-09-07)<br>　　claims, examples | 1-33 |
| Y | GHOSH, D. et al. Leveraging Multilayered "Omics" Data for Atopic Dermatitis: A Road Map to Precision Medicine. Frontiers in Immunology. 12 December 2018, vol. 9, Article 2727, pp. 1-22<br>　　abstract, p. 6, left column, paragraph [0003] to p. 7, right column, paragraph [0002], p. 16, left column, paragraph [0002] to right column, paragraph [0001], fig. 2, 4 | 1-33 |
| A | JP 2005-110602 A (SUMITOMO PHARMACEUT CO LTD) 28 April 2005 (2005-04-28)<br>　　examples 1-3 | 1-33 |
| A | JP 2015-227865 A (KAO CORP) 17 December 2015 (2015-12-17)<br>　　examples | 1-33 |

Form PCT/ISA/210 (second sheet) (January 2015)

**INTERNATIONAL SEARCH REPORT**
Information on patent family members

International application No.

**PCT/JP2021/043714**

| Patent document cited in search report | | | Publication date (day/month/year) | Patent family member(s) | | | Publication date (day/month/year) |
|---|---|---|---|---|---|---|---|
| KR | 10-1818672 | B1 | 16 January 2018 | (Family: none) | | | |
| JP | 2020-074769 | A | 21 May 2020 | CN 112955551 A claims, paragraphs [0029], [0046]-[0082], test examples 3, 6, tables 7-1 to 7-24 WO 2020/091044 A1 | | | |
| JP | 2020-520938 | A | 16 July 2020 | US 2018/0333382 A1 paragraph [0094] WO 2018/211325 A1 CN 110996937 A | | | |
| JP | 2017-198632 | A | 02 November 2017 | US 2019/0145967 A1 paragraphs [0004]-[0005] WO 2017/188101 A1 EP 3450978 A1 CN 109073640 A KR 10-2019-0004269 A | | | |
| WO | 2018/161062 | A1 | 07 September 2018 | JP 2020-508689 A claims, examples 1-3 EP 3589213 A1 | | | |
| JP | 2005-110602 | A | 28 April 2005 | (Family: none) | | | |
| JP | 2015-227865 | A | 17 December 2015 | US 2017/0192015 A1 examples WO 2015/170781 A1 CN 106460031 A | | | |

Form PCT/ISA/210 (patent family annex) (January 2015)

**EP 4 253 547 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018008319 A
- JP 2020074769 A
- JP 2018023756 A

**Non-patent literature cited in the description**

- **KATO et al.** *The Japanese Journal of Dermatology,* 2018, vol. 128, 2431-2502
- **LIN et al.** *Adv. Ther.,* 2017, vol. 34, 2601-2611
- **SUGAWARA et al.** *Allergy,* 2002, vol. 57, 180-181
- **OHTA et al.** *Ann. Clin. Biochem.,* 2012, vol. 49, 277-284
- Japanese Dermatological Association. *Journal of Dermatology,* 2018, vol. 128 (12), 2431-2502
- **DAWSON et al.** *Phys. Med. Biol.,* 1980, vol. 25
- *PNAS,* 2003, vol. 100, 12271-12276
- *Biol. Reprod.,* 1998, vol. 58, 302-311
- **J. SAMBROOK et al.** Molecular Cloning: A Laboratory Manual. Cold Spring Harbor Laboratory Press, 2001
- **LOVE M.I. et al.** *Genome Biol,* 2014
- **HANIFIN et al.** *Exp. Dermatol.,* 2001, vol. 10
- **CHARMAN et al.** *Arch. Dermatol.,* 2004, vol. 140